# EUROPEAN PATENT APPLICATION

(11) **EP 2 433 584 A1**
(43) Date of publication of application: **28.03.2012**
(21) Application number: 09844835.0
(22) Date of filing: 22.05.2009
(51) Int. Cl.: A61B 18/22

(54) **FIBRE OPTIC CABLE MODIFIED AT ONE OF THE ENDS THEREOF PROVIDED WITH MEANS FOR VENTILATION OF SAID ENDS**

(71) Applicant: Intermedic Arfran, S.a., 08029 Barcelona (ES); Advanced Fiber Tools GmbH, 09648 Mittweida (DE)
(72) Inventor: ARCUSA VILLACAMPA, Francisco, Javier, E-08029 Barcelona (ES)
(74) Representative: Morgades y Manonelles, Juan Antonio
(86) International application number: PCT/ES2009/000285
(87) International publication number: WO 2010/133712

(57) **Abstract**

The invention refers to the modification of one of the ends of an optical fibre of the type used together with laser radiation generators for medical/surgical applications. This novel type of fibre may be used with the aid of an endoscope, cytoscope, fetoscope, etc., for cutting, coagulating and vaporizing soft tissue.

## Description

### Object of the Invention.

More specifically, the invention refers to the modification of one of the ends of an optical fibre of the type used together with laser radiation generators for medical/surgical applications. This novel type of fibre may be used with the aid of an endoscope, cytoscope, fetoscope, etc., for cutting, coagulating and vaporizing soft tissue.

### State of the art.

In this technical sector, it is a collection of laser generation devices, applied by the corresponding accessories, and with the aid of a vision system (endoscope, cytoscope, fetoscope, etc.) taking laser energy inside the human body that is being treated medically.

The suitability of this collection of devices depends on several factors, some inseparable from the features of the laser generator itself, others on the type of fibre optic cable most suitable for the range of energy, others on the manner in which this energy is transmitted along the fibre optic cable and finally on the features of the end of this fibre optic cable.

Within this group of devices, the invention is for use with laser radiation generators, through a fibre optic cable aided with a vision system, the tip of which or distal end is adapted for prostate treatment by the application of the energy emitted by this tip to cut, coagulate or vaporise mainly benign prostatic hyperplasia or for other tissues needing treatment.

The tip of this optical fibre cable measuring 2 to 5 metres, is finished at an angle with regard to the longitudinal axis of the fibre so it can emit the energy in an oblique manner and is visible from the vision system.

The corresponding laser radiations are emitted from this angled end with regard to the longitudinal axis, forming a cone shaped beam that widens as it distances itself from the end, therefore, the application of energy is not precise enough, and in some instances burns areas of tissue around those we wish to eliminate specifically. This divergence is called numerical opening and depends on the materials used to make the fibre optic cable.

### Scope of the Invention.

To improve the precision of the laser energy emitted by the end the of the fibre optic cable, modifying its configuration, divergence to achieve a collimated beam, convergent or divergent according to the desired action on the tissues.

Another aim of the invention is to increase the surface output and hence decrease the power density at the output point. Thus reducing the adverse effect of the carbonisation of tissues that could adhere to the fibre cable outlet.

What is achieved by shaping the distal end of the optical fibre is equivalent to incorporating a lens. And according to the shape given to the end of the fibre, different effects are achieved. For example:
- Focus: improve the vaporization capacity.
- Collimate: makes the laser beam at the output parallel and hence, the effect on the tissues will not vary according to the distance from which the tissue is treated.
- Diverge: to unfocus the beam and increase the heating effect (coagulation) reducing the cutting effect.

Additionally, a flow of water, saline solution or other liquid can be added, flowing from the front of the fibre optic output to cool it and thus avoid the adherence of tissue remains. This airflow or pressurised water may be generated from the laser device or by any other external element, which in turn will be modified; thereby it is the object of the invention so that it includes a means of generating pressure.

### Description of the Invention.

The invention will be applied to fibre optic cables between 200 and 1,000 um diameter and to be used with laser generators in the 400 nm to 3,000 nm wavelength.

The shape of the end of the fibre optic cable will be considerably curved, and the cover sheath will reach the diametric plane of the end, facilitating the transmission of the energy in an inverse cone beam a, namely that it diminishes its width as it distances itself from the end.

The nature of the fibre optic cables used in this invention will be, for example (although not exclusively) the following:
- Core: silica from 200 microns to 1,000 microns.
- Design of fibre: pure fused silica core
- Coating of fused silica doped with fluorine, pure silica, fluoropolymer, etc.

### - Coatings:

- Acrylate coating (- 40°C to 85°C).
- Silicone resin coating (- 40°C to 180°C).
- Polyamide acrylate coating (- 190°C to 385°C).

### - Covers:

- Nylon (- 40°C to 100°C).
- ETFE (- 200°C to 150°C).
- Acrylate (- 40°C to 85°C).
- TEFZEL

### - Options:

- Core/ coating ratio: 1.05, 1.07, 1,15, 1.20, 1.30, 1.40.
- Numerical openings 0.07 to 0.28.
- Metal cover (-190°C to 750° C).
- Connectors (MA, FC/PC, ST, DIN).
- AS - Fibre cables.

Another of the aims of the invention is the covering of this end part with an encapsulation open at its upper edge and able to direct a stream of pressurised air to help with the carbonization of tissues that could adhere to this end part or tip.

Other details and characteristics shall be shown throughout the description below referring to drawings attached to this report which are shown for illustrative but not limiting purposes only in an embodiment of the invention.

Below is a numbered list for the different parts of the invention, which are indicated in the attached drawings: (10) end of the fibre optic cable (11), (11) fibre optic cable, (12) plane at the end (11), (13) cover, (14) upper outline of the cover, (15 - 15') diametric plan, (16) outline, (17) direct conical beam, (18) inverse conical beam, (19) apex of the inverse conical beam (18), (19) area of application, (20) encapsulation, (21) top edge of (20), (22) laminar flow,

### Description of the drawings.

Figure 1 is a side elevation view of the end part (10) of a fibre optic cable (11), in an embodiment according to the current state of the art.
Figure 2 is a section of the end part (10) of a fibre optic cable (11), in one of the embodiments of the invention.
Figure 3 is a section of the end part (10), of a fibre optic cable (11), in one of the embodiments of the invention in which in the vicinity of the cover (13) there is an encapsulation (20).
Figure 4 is a longitudinal sectional view of part of prostate or similar tissue (20) inside which, and with the aid of a cytoscope (22) a fibre optic cable is inserted and directed and its end (10).

### Description of a preferred embodiment of the invention.

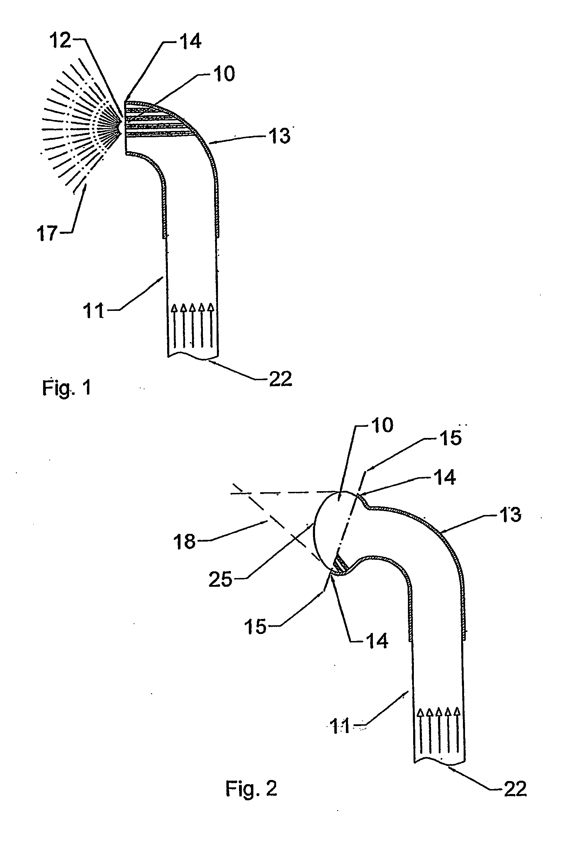

As shown in the figure 1, a conventional embodiment and forming part of the state of the art is a fibre optic cable (11), whose end is a plane perpendicular (12) to the cover (13), this only exists at the tip or end part and in its vicinity, with the light circulating in the direction of the arrows (22), obtained from a laser energy generator not included in the drawings. The energy reaches the end (10) of the cable (11), and is emitted in the form of a cone-shaped divergent light beam (17), to the area of application inside the human body where it is desired to eliminate human tissue by burning, it will subsequently regenerate.

The fibre optic cable (11) is directed by means of a conventional cytoscope not shown in the drawing, through the inside of the passage or cavity (21) where the user wishes to burn and remove tissue by means of the cone of energy (17), however, according to the distance of the plane (12) of the end parte (10) from the area (21) of tissue to be burnt, as the laser firing control is in the vicinity of the laser generator, far from the tip (12), it is difficult to know the precision of the cone applied (17), and therefore the area burnt is either more or less than the desired area, and the accuracy of the surgical operation is significantly decreased.

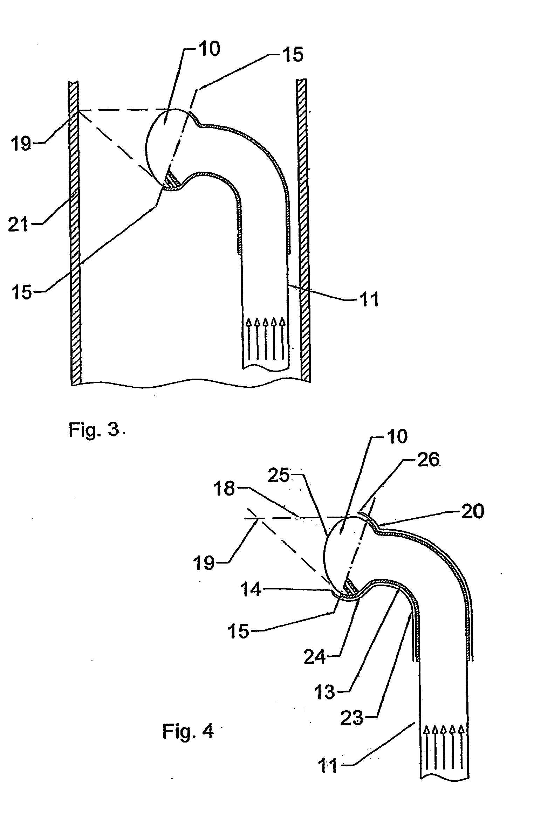

The invention described changes the configuration of the end part (10) so that instead of a flat surface (12), as can be seen in figure 1, it presents a curved surface, as can be seen in figure 2, 3 and 4, as a surface (25), this curved configuration generates a convergent cone (18) with apex (19), that ensures precision in the application of laser energy to burn the desired areas (21).

Another of the aims of the invention is to provide this end with a means of irrigation, such as an encapsulation (21) concentric to the cover (13) so that between the wall (23) of the same and the outer wall (24) of the cover (13) a current of air circulates and exits from the edge (21), this laminar flow can be delivered by the doctor, operating the device from the generator of laser energy itself to the end (10) of the fibre optic cable (11), with the corresponding control, detaching from the surface (24) of (10), and burnt tissue material that may have adhered to the surface (24) and thus avoiding its carbonization and consequent loss of power flowing from this surface (24).

The irrigation option will consist in an external jacket around the fibre, through which gas or liquid can flow with the aim of cooling the tip of the fibre (so it can transmit more power without deteriorating) and also to avoid the adherence of tissue remains.

Having sufficiently described this invention using the figures attached, it is easy to understand that any modification may be made to the detail which may be deemed to be appropriate, whenever these changes do not alter the essence of the invention summarised in the following claims.

## Claims

1. **- "FIBRE OPTIC CABLE MODIFIED AT ONE OF ITS ENDS, PROVIDED WITH A MEANS FOR VENTILATION OF SAID ENDS"** used for transmission and orientation from a laser generator to the point of application of this energy in the area of tissue the user wishes to eliminate, directing and moving said cable, by means of a cytoscope inside which it moves, until its end, protected by a cover, is in the vicinity of the area to be burnt, **characterised in that** the fibre optic cable (11) transmitting the energy, generated by a laser energy generator for application at a distance, has at its end (10) a curved surface (25) from which the laser energy is emitted in an inverted conical beam (18) with apex (19), this end (10) being protected with a cover (13) which comes from the rear to the diametrical plane (15-15') .

2. **- "FIBRE OPTIC CABLE MODIFIED AT ONE OF ITS ENDS, PROVIDED WITH A MEANS FOR VENTILATION OF SAID ENDS"** according to claim 1, **characterised in that** in an alternative version of the invention, the cable (11) has on this end a means of irrigation, such as an encapsulation (21) concentric to the cover (13) so that between the wall (23) of the same and the outer wall (24) of the cover (13) a current of air circulates and exits from the edge (21) as laminar flow (26).

3. **- "FIBRE OPTIC CABLE MODIFIED AT ONE OF ITS ENDS, PROVIDED WITH A MEANS FOR VENTILATION OF SAID ENDS"** according to preceding claims, **characterised in that** the irrigation option is achieved by means of an encapsulation (20), through which gas or liquid can flow with the aim of cooling the tip of the fibre (so it can transmit more power without deteriorating) and also to avoid the adherence of tissue remains.
